# EUROPÄISCHE PATENTSCHRIFT

(11) **EP 1 792 951 B1**
(45) Veröffentlichungstag und Bekanntmachung des Hinweises auf die Patenterteilung: **21.12.2011**
(21) Anmeldenummer: 06021893.0
(22) Anmeldetag: 19.10.2006
(51) Int. Cl.: C09B 69/10, A61Q 5/10, A61K 8/49, A61Q 17/04

(54) **Biomimetische Melaninpigmente**
Biomimetic melanine pigments
Pigments mélaniques biomimétiques

(30) Priorität: 29.11.2005 DE 102005057183
(43) Veröffentlichungstag der Anmeldung: 06.06.2007
(73) Patentinhaber: Henkel AG & Co. KGaA, 40589 Düsseldorf (DE)
(72) Erfinder: Knübel, Georg, 40219 Düsseldorf (DE); Döring, Thomas, 41542 Dormagen (DE); Schulze zur Wiesche, Erik, 20251 Hamburg (DE); Benicke, Wolfgang, 40723 Hilden (DE); Höffkes, Horst, 40595 Düsseldorf (DE)

(56) Entgegenhaltungen:
- EP-B- 0 530 229
- US-A- 3 993 436
- US-A- 5 227 459
- US-A- 5 346 509

## Beschreibung

Die vorliegende Erfindung betrifft biomimetische Melaninpigmente, deren Herstellung sowie deren Einsatz in Färbe- und UV-Schutzmitteln als Farbstoff, UV-Lichtschutzfilter und Antioxidans.

Menschliche Haut und Haare werden heute in vielfältiger Weise mit kosmetischen Zubereitungen behandelt. Dazu gehören etwa die Reinigung der Haut bzw. der Haare mit Shampoos, die Pflege und Regeneration mit Cremes, Spülungen und Kuren sowie das Bleichen und Färben von Haut und Haaren mit Schminke, Färbemitteln und Tönungsmitteln. Dabei spielen Mittel zur Veränderung oder Nuancierung der Farbe der Gesichtshaut, der Wimpern und des Kopfhaares eine herausragende Rolle.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, vom sogenannten Entwickler-Typ (Entwicklerkomponente) und Kuppler-Typ (Kupplerkomponente). Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxiidationsfärbemittel zeichnen sich durch hervorragende, lang anhaltende Färbeergebnisse aus.

Schließlich hat ein weiteres oxidatives Färbeverfahren große Beachtung gefunden. Bei diesem Verfahren werden Vorstufen des natürlichen Haarfarbstoffes Melanin auf das Substrat aufgebracht; diese bilden dann im Rahmen oxidativer Prozesse naturanaloge Farbstoffe aus. Ein solches Verfahren mit 5,6-Dihydroxyindolin als Farbstoffvorprodukt wurde in der EP-B1-530 229 beschrieben.

Für temporäre Färbungen werden üblicherweise Färbe- oder Tönungsmittel verwendet, die als färbende Komponente sogenannte Direktzieher enthalten. Hierbei handelt es sich um weitestgehend lösliche Farbstoffmoleküle, die direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Waschen bzw. Shampoonieren in der Regel empfindlich, so dass eine vielfach unerwünschte Nuancenverschiebung oder gar eine sichtbare "Entfärbung" eintreten kann.

Insbesondere als Schminke für Haut und für Wimpern finden kosmetische Mittel Anwendung, die unlösliche Farbpigmente enthalten. Meist handelt es sich dabei um anorganische Farbpigmente wie Eisenoxidgelb (α-FeOOH; Goethit), Eisenoxidrot (α-Fe₂O₃; Hämatit), Eisenoxidschwarz (Fe₃O₄; Magnetit) oder auf Titandioxid basierende Pigmente. Diese können zur besseren Einarbeitbarkeit in das Trägermedium hydrophil oder hydrophob beschichtet sein.

Als weitere Möglichkeit kommt die Verwendung organischer Farbpigmente, beispielsweise synthetisch zugänglicher Melaninpigmente, als farbgebende Komponente für Schminken in Betracht. Die aus einer oxidativen Kupplung von 5,6-Dihydroxyindolin herstellbaren naturanalogen Farbstoffe sind unlösliche naturverwandte Melaninfarbpigmente, welche sich ohne Anwesenheit von 5,6-Dihydroxyindolin, wie in der Druckschrift WO-A1-93/13744 offenbart, zur temporären Färbung eignen.

Gemäß G. Prota et al., Gazz. Chim. Italiana, 1993, 123, 241-242 können Melaninpigmente durch Oxidation von 3,4-Dihydroxyphenylalanin (DOPA) synthetisch gewonnen werden. Allerdings sind diese Pigmente chemisch instabil.

Nachteile der synthetisch hergestellten, naturanalogen Melaninpigmente liegen in deren Unlöslichkeit. Pawelek et al. (US-A-5 227 459) beschreiben die nicht enzymatische Herstellung von löslichen Melaninpigmenten aus 5,6-Dihydroxyindol-2-carbonsäure (DHICA). Durch die teure Herstellung des Ausgangsstoffs DHICA erscheint die Anwendung der Melaninpigmente gemäß Herstellverfahren nach Pawelek als unwirtschaftlich.

Aufgabe der vorliegenden Erfindung ist es daher, einen Herstellungsprozess biomimetischer Melaninpigmente bereitzustellen, durch den eine wirtschaftliche Anwendung von biomimetischen Melaninpigmenten ermöglicht wird.

Es wurde überraschenderweise gefunden, dass das wohlfeile 5,6-Dihydroxyindolin bzw. dessen Derivate sich gemeinsam mit definierten, wohlfeilen Phenylalanin-Derivaten zu löslichen Melaninpigmenten umsetzen lassen.

Ein erster Gegenstand der Erfindung ist daher ein Verfahren zur Herstellung biomimetischer Melaninpigmente, in dem in einem Reaktionsmedium mindestens ein Indolinderivat, ausgewählt aus 5,6-Dihydroxyindolin bzw. deren physiologisch verträglichen Salzen, gemeinsam mit mindestens einem Phenylalaninderivat, ausgewählt aus 3,4-Dihydroxyphenylalanin (DOPA) bzw. dessen physiologisch verträgliches Salz, in Gegenwart mindestens eines Oxidationsmittels umgesetzt wird.

Da es sich bei dem erfindungsgemäßen Indolinderivat 5,6-Dihydroxyindolin um eine Amino-Verbindung handelt, lassen sich aus diesen in üblicher Weise die bekannten Säureadditionssalze herstellen. Alle Aussagen dieser Schrift und demgemäß der beanspruchte Schutzbereich beziehen sich daher sowohl auf die in freier Form vorliegenden Verbindungen als auch auf deren wasserlösliche, physiologisch verträgliche Salze. Beispiele für solche Salze sind die Hydrochloride, die Hydrobromide, die Sulfate, die Phosphate, die Acetate, die Propionate, die Citrate und die Lactate. Die Hydrochloride und die Sulfate sind dabei besonders bevorzugt.

Säureadditionssalze lassen sich ebenso von dem Phenylalaninderivat 3,4-Dihydroxyphenylalanin bilden. Darüber hinaus lässt sich durch Einsatz von Basen, z.B. von Alkalihydroxiden, das Proton der Carboxylgruppe unter Bildung von Metallsalzen abstrahieren. Die Metallsalze besitzen physiologisch verträgliche Kationen wie Alkali-, Erdalkali-, Ammonium- oder Zinkionen. Ferner liegt bei neutralem pH-Wert 3,4-Dihydroxyphenylalanin überwiegend in einer zwitterionischen Form vor.

Das Indolinderivat und das Phenylalaninderivat werden bevorzugt in einem Molverhältnisbereich von 2 zu 3 bis 9 zu 1, besonders bevorzugt von 4 zu 1 bis 3 zu 2, in dem erfindungsgemäßen Verfahren eingesetzt.

Das Reaktionsmedium ist bevorzugt ein wässriges Medium. Darüber hinaus sind ebenfalls wässrige Lösemittelgemische mit in Wasser löslichen organischen Lösemitteln, wie beispielsweise Ethanol, Isopropanol oder Aceton, geeignet.

Der pH-Wert des Reaktionsmediums beträgt bevorzugt während des Verfahrens pH 7 bis pH 12, besonders bevorzugt pH 8 bis pH 10.

Das Reaktionsmedium enthält zur Einstellung des pH-Werts zusätzlich mindestens ein Alkalisierungsmittel. Als geeignete Alkalisierungsmittel haben sich Ammoniak, Ammonium-, Alkalimetall- und Erdalkalimetallhydroxide, Ammonium-, Alkalimetall- und Erdalkalimetallcarbonate, Ammonium-, Alkalimetall- und Erdalkalimetallhydrogencarbonate, Erdalkalimetallhydroxycarbonate und Ammonium-, Alkalimetall- und Erdalkalimetallcarbamide, sowie Alkaliphosphate und Alkalimetasilikate erwiesen. Weiterhin geeignet sind organische Aminverbindungen, wie beispielsweise Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol oder Triethanolamin. Da sich der pH-Wert während der Reaktionsdauer ändern kann, ist es vorteilhaft eine Puffersubstanz zuzusetzen. Ohne Einsatz von Puffersubstanzen kann der pH-Wert über die Reaktionsdauer überwacht und manuell oder automatisch reguliert werden.

Das im erfindungsgemäßen Verfahren verwendete Oxidationsmittel muß in der Lage sein, das Indolinderivat-zu oxidieren. Zu diesem Zweck eignet sich besonders Sauerstoff, Metallperoxide (wie Magnesiumperoxid oder Bariumperoxid), Persäuren wie Wasserstoffperoxid oder organische Percarbonsäuren, sowie Peroxoverbindungen, wie z.B. Peroxidisulfate oder Perborate. Bevorzugt wird als Oxidationsmittel Sauerstoffgas oder sauerstoffgashaltige Gasgemische, wie beispielsweise Luft, eingesetzt. Zu diesem Zweck kann das Reaktionsgemisch in einem offenen Gefäß an der Luft gerührt werden. Ebenso kann das sauerstoffgashaltige Gasgemisch bzw. der Sauerstoff direkt in die Reaktionsmischung hineingeblasen werden.

Die Reaktionszeit beträgt bevorzugt 12 bis 72 Stunden, besonders bevorzugt 24 bis 48 Stunden. Es kann bevorzugt sein, eine Portionierung des Indolinderivats vorzunehmen. Dabei werden zu Beginn der Reaktion zunächst die Reaktanden aus Indolinderivat, ausgewählt aus 5,6-Dihydroxyindolin bzw. deren physiologisch verträglichen Salzen, und Phenylalaninderivat, ausgewählt aus 3,4-Dihydroxyphenylalanin (DOPA) bzw. dessen physiologisch verträgliches Salz, im Molverhältnis von 1 zu 1.5 bis 1.5 zu 1, insbesondere von 1 zu 1, eingesetzt. Nach Ablauf eines Drittels bis Zweidrittel der geplanten Reaktionszeit wird eine weitere Menge an Indolinderivat zugesetzt, bevorzugt die 0.5-fache bis 1.5-fache Menge des Phenylalaninderivats.

Nach Ablauf der Reaktionszeit ist es bevorzugt, das Reaktionsgemisch auf einen pH-Wert von pH < 7, insbesondere auf einen pH-Wert von pH 2 bis 5 einzustellen.

Ein zweiter Gegenstand der Erfindung sind biomimetische Melaninpigmente, die nach dem Verfahren des ersten Erfindungsgegenstandes hergestellt wurden.

Ein dritter Gegenstand der Erfindung sind kosmetische Mittel, die in einem kosmetischen Träger biomimetische Melaninpigmente enthalten, welche nach dem Verfahren des ersten Erfindungsgegenstandes hergestellt wurden.

Wenn die erfindungsgemäßen Mittel als Färbemittel verwendet werden sollen, kann es vorteilhaft sein, zusätzlich mindestens eine weitere, d.h. von den erfindungsgemäßen Melaninpigmenten verschiedene, farbverändernde Komponente zuzufügen. Die zusätzliche farbverändernde Komponente wird wiederum bevorzugt ausgewählt
a) aus mindestens einem Oxidationsfarbstoffvorprodukt vom Typ der Entwicklerkomponenten und gegebenenfalls zusätzlich mindestens einer Kupplerkomponente
   und/oder
b) aus Oxofarbstoffvorprodukten
   und/oder
c) aus mindestens einem direktziehenden Farbstoff.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterozyklische Hydrazone, 4-Aminopyrazolderivate sowie 2,4,5,6-Tetraaminopyrimidin und dessen Derivate eingesetzt.

Besonders bevorzugte p-Phenylendiamine sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)amino-2-methylanilin, 4-N,N-Bis-(β-hydroxyethyl)amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen. Erfindungsgemäß ganz besonders bevorzugte p-Phenylendiaminderivate sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Bevorzugte zweikernige Entwicklerkomponenten sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze. Ganz besonders bevorzugte zweikernige Entwicklerkomponenten sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetra-oxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen.

Bevorzugte p-Aminophenole sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethylphenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterozyklischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)amino-3-amino-pyridin, 2,3-Diamino-6-methoxy-pyridin, 2-(β-Methoxyethyl)amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(□-aminoethyl)amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)amino-1-methylpyrazol.

Als Kupplerkomponenten werden in der Regel m-Phenylendiaminderivate, Naphthole, Resorcin und Resorcinderivate, Pyrazolone und m-Aminophenolderivate sowie heterozyklische Verbindungen verwendet.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,

- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin und die physiologisch verträglichen Salzen der vorgenannten Verbindungen.

Die kosmetischen Mittel des erfindungsgemäßen Verfahrens enthalten die Entwicklerkomponenten bevorzugt in einer Menge von 0,005 bis 10 Gew.-%, vorzugsweise von 0,1 bis 5 Gew.-%, jeweils bezogen auf das gesamte Mittel.

Die kosmetischen Mittel des erfindungsgemäßen Verfahrens enthalten die Kupplerkomponenten bevorzugt in einer Menge von 0,005 bis 10 Gew.-%, vorzugsweise von 0,1 bis 5 Gew.-%, jeweils bezogen auf das gesamte Mittel.

Das erfindungsgemäße Mittel kann als farbverändernde Komponente in Form der Oxofarbstoffvorprodukte mindestens eine Kombination, aus mindestens einer Verbindung der Komponente
A Verbindungen, die eine reaktive Carbonylgruppe enthalten mit mindestens einer Verbindung der Komponente
B Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen
enthalten.

Erfindungsgemäße Verbindungen mit einer reaktiven Carbonylgruppe (im Folgenden auch reaktive Carbonylverbindungen oder Komponente A genannt) besitzen mindestens eine Carbonylgruppe als reaktive Gruppe, welche mit den Verbindungen der Komponente B unter Ausbildung einer beide Komponenten verknüpfenden chemischen Bindung reagiert. Ferner sind erfindungsgemäß auch solche Verbindungen als Komponente A umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente B stets vorhanden ist. Diese Derivate sind bevorzugt Kondensationsverbindungen von reaktiven Carbonylverbindungen mit
i) Aminen und deren Derivate unter Bildung von Iminen oder Oximen als Kondensationsverbindung
ii) Alkoholen unter Bildung von Acetalen oder Ketalen als Kondensationsverbindung
iii) Wasser unter Bildung von Hydraten als Kondensationsverbindung von Aldehyden.

Die Komponente A wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxyacetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxybenzaldehyd, 4-Hydroxy-3,5-dimethyl-benzaldehyd, 3,5-Diethoxy-4-hydroxybenzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxybenzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 2-Methoxy-zimtaldehyd, 4-Methoxy-zimtaldehyd, 4-Hydroxy-3-methoxy-zimtaldehyd, 3,5-Dimethoxy-4-hydroxyzimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylaminobenzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und lmidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon, Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-Salze, bevorzugt mit Benzolsulfonat, p-Toluolsulfonat, Methansulfonat, Perchlorat, Sulfat, Chlorid, Bromid, Iodid, Tetrachlorozinkat, Methylsulfat, Trifluormethansulfonat oder Tetrafluoroborat als Gegenion, Isatin, 1-Methyl-isatin, 1-Allylisatin, 1-Hydroxymethyl-isatin, 5-Chlor-isatin, 5-Methoxy-isatin, 5-Nitroisatin, 6-Nitroisatin, 5-Sulfo-isatin, 5-Carboxy-isatin, Chinisatin, 1-Methylchinisatin, sowie beliebigen Gemischen der voranstehenden Verbindungen.

Als CH-acide werden im allgemeinen solche Verbindungen angesehen, die ein an ein aliphatisches Kohlenstoffatom gebundenes Wasserstoffatom tragen, wobei aufgrund von elektronenziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quaternierten N-Heterozyklen mit einer in Konjugation zum quartären Stickstoff stehenden CH-aciden Alkylgruppe entstehen.

Die CH-aciden Verbindungen der Komponente B sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methylbenzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Bevorzugte primäre oder sekundäre aromatische Amine der Komponente B sind ausgewählt aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxybenzoesäure, 4-Amino-3-hydroxybenzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlorbenzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methylpyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

Die Verbindungen der Komponente A und die Verbindungen der Komponente B werden vorzugsweise in den kosmetischen Mitteln jeweils in einer Menge von 0,03 bis 65 mmol, insbesondere von 1 bis 40 mmol, bezogen auf 100 g des gesamten Nuanciermittels, verwendet. Das molare Verhältnis von der Verbindung der Komponente A und der Verbindung der Komponente B kann im Bereich von 0,5 bis 2,0 liegen, wobei vorzugsweise äquimolare Mengen eingesetzt werden. Das anwendungsbereite Mittel wird bei getrennter Lagerung der Komponenten A und B unmittelbar vor der Anwendung durch Mischen hergestellt.

Bevorzugte direktziehende Farbstoffe, die in den kosmetischen Mitteln als farbverändernde Komponente Verwendung finden, sind Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die kosmetischen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Die Verbindungen, die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen kosmetischen Mittel können die direktziehenden Farbstoffe bevorzugt in einer Menge von 0,01 bis 20 Gew.-%, bezogen auf das anwendungsbereite Mittel enthalten.

Weiterhin können die kosmetischen Mittel auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den kosmetischen Mitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel können neben den erfindungsgemäßen Melaninpigmenten zusätzlich mindestens einen weiteren UV-Lichtschutzfilter enthalten. Unter UV-Lichtschutzfiltern sind organische Substanzen zu verstehen, die in der Lage sind, ultra-violette Strahlen zu absorbieren und die aufgenommene Energie in Form längerwelliger Strahlung, z.B. Wärme wieder abzugeben.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)-propan-1,3-dion.

Als UV-B-Filter eignen sich beispielsweise die nachfolgenden Substanzen. In diesem Zusammenhang sind als öllösliche Substanzen zu nennen:
- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion;

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sul-fonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Die UV-A und UV-B-Filter können selbstverständlich auch in Mischungen eingesetzt werden.

Neben den genannten löslichen Stoffen kommen auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Die erfindungsgemäßen Mittel können zusätzlich mindestens ein Oxidationsmittel enthalten. Als Oxidationsmittel kommt insbesondere Wasserstoffperoxid und/oder mindestens ein Anlagerungsprodukt davon, insbesondere an anorganische oder organische Verbindungen, wie beispielsweise Natriumperborat, Natriumpercarbonat, Magnesiumpercarbonat, Natriumpercarbamid, Polyvinylpyrrolidon-n H₂O₂ (n ist eine positive ganze Zahl größer 0), Harnstoffperoxid und Melaminperoxid in Frage.

Der pH-Wert der erfindungsgemäßen kosmetischen Mittel liegt bevorzugt in dem pH-Wertbereich von pH x bis pH y, besonders bevorzugt von pH xx bis pH yy.

Die kosmetischen Mittel enthalten bevorzugt mindestens ein Alkalisierungsmittel, wie beispielsweise Ammonium-, Alkalimetall- und Erdalkalimetallhydroxide, -carbonate, - hydrogencarbonate, -hydroxycarbonate, -metasilikate oder -carbamide, sowie Alkaliphosphate oder Alkanolamine, insbesondere primäre Amine mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Das erfindungsgemäße kosmetische Mittel enthält die erfindungsgemäßen Melaninpigmente in einem kosmetischen Träger. Als kosmetische Träger eignen sich besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die kosmetischen Träger können insbesondere wässrig oder wässrig-alkoholisch sein.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

Die erfindungsgemäßen Mittel können weiterhin alle für solche Zubereitungen bekannten Wirk-, Zusatz- und Hilfsstoffe enthalten. In vielen Fällen enthalten die erfindungsgemäßen Mittel zusätzlich mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß können als kationische Tenside insbesondere solche vom Typ der quartären Ammoniumverbindungen, der Esterquats und der Amidoamine eingesetzt werden.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Marken Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Marke Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Ferner können die erfindungsgemäßen Mittel weitere Wirk-, Hilfs- und Zusatzstoffe, wie beispielsweise
- nichtionische Polymere wie beispielsweise VinylpyrrolidonNinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidon-Copolymere, Vinylpyrrolidon-Imidazolinium-methochlorid-Copolymere und quaternierter Polyvinylalkohol,
- zwitterionische und amphotere Polymere wie beispielsweise Acrylamidopropyltrimethylammoniumchlorid/Acrylat-Copolymere und Octylacrylamid/Methylmethacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butylacrylamid-Terpolymere,
- amphiphile Polymere, wie beispielsweise die Polymere gemäß der INCI-Bezeichnung:Bezeichnungen Acrylates/Beheneth-25 Methacrylate Copolymer, Acrylates/C10-30 Alkyl Acrylate Crosspolymer, Acrylates/Ceteth-20 Itaconate Copolymer, Acrylates/Ceteth-20 Methacrylate Copolymer, Acrylates/Laureth-25 Methacrylate Copolymer, Acrylates/Palmeth-25 Acrylate Copolymer, Acrylates/Palmeth-25 Itaconate Copolymer, Acrylates/Steareth-50 Acrylate Copolymer, Acrylates/Steareth-20 Itaconate Copolymer, Acrylates/Steareth-20 Methacrylate Copolymer, Acrylates/Stearyl Methacrylate Copolymer, Acrytates/Vinyl Isodecanoate Crosspolymer,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein vierter Gegenstand ist die Verwendung der Melaninpigmente des zweiten Erfindungsgegenstandes als UV-Lichtschutzfilter.

Ein fünfter Gegenstand der Erfindung ist die Verwendung der Melaninpigmente des zweiten Erfindungsgegenstandes als farbverändernde Komponente bzw. als Farbstoff zum Färben keratinhaltiger Fasern, insbesondere menschlicher Haut oder Humanhaar.

Ein sechster Gegenstand der Erfindung ist die Verwendung der Melaninpigmente des zweiten Erfindungsgegenstandes als Antioxidans. Insbesondere schützen die besagten Melaninpigmente in ihrer Funktion als Antioxidans ein Substrat, wie z.B. die Haut und/oder die Haare, vor oxidativem Stress. Der oxidative Stress wird beispielsweise durch freie Radikale verursacht.

Für die Erfindungsgegenstände zwei bis sieben gilt mutatis mutandis das für den ersten Erfindungsgegenstand Gesagte.

### Beispiele

### 1. Herstellung erfindungsgemäßer biomimetischer Melaninpigmente

80,6 g Ammoniumsulfat wurden in 3 Liter Wasser gelöst und mit konzentrierter wässriger Ammoniaklösung auf pH 9 eingestellt. Im Anschluss wurden 22,8 g DOPA und 26,8 g 5,6-Dihydroxyindolin-hydrobromid, gelöst in 160 mL Wasser, zugegeben. Der pH-Wert wurde durch Zugabe konzentrierter wässriger Ammoniaklösung auf pH 9 gehalten. Das Reaktionsgemisch wurde 22 Stunden im offenen Becherglas gerührt und dabei über eine D1-Fritte Luft in die Reaktionsmischung eingeleitet. Anschließend wurden weitere 26,8 g 5,6-Dihydroxyindolin-hydrobromid in 160 mL Wasser gelöst zugegeben, der pH-Wert erneut auf pH 9 eingestellt und für weitere 22 h unter o.g. Lufteinleitung rühren gelassen. Im Anschluss wurde mit konzentrierter Schwefelsäure auf pH 2,5 angesäuert. Das angefallene Melaninpigment wurde abfiltriert und zweimal mit Wasser und abschließend mit Ethanol gewaschen. Nach Trocknung im Vakuum erhielt man 55,5 g des erfindungsgemäßen Melaninpigments.

### 2. Kosmetische Mittel

Es wurden nachfolgende erfindungsgemäße kosmetische Mittel bereitgestellt. Dabei wurden als biomimetische Melaninpigmente stets Pigmente verwendet, die gemäß Herstellvorschrift unter 1. hergestellt wurden.

Alle nachfolgenden Mengenangaben sind falls nicht anders gekennzeichnet Gewichtsprozent (Gew.-%), bezogen auf das Gewicht des kosmetischen Mittels.

Nachfolgende handelsübliche Rohstoffe wurden zur Formulierung der kosmetischen Mittel verwendet:

| | | |
|---|---|---|
| Handelsname | INCI-Bezeichnung | Lieferant/Hersteller |
| Abil Quat^{®} 3270 | Quaternium-80 | Goldschmidt |
| Baysilon^{®} M350 | Silikonöl | Bayer |
| Brij^{®} 30 | Laureth-4 | Uniquema |
| Cetiol^{®} SB 45 | Butyrospermum Parkii (Shea butter) | Cognis |
| Cetiol^{®} 868 | Ethylhexyl Stearate | Cognis |
| Cetiol^{®} HE | PEG-7 Glyceryl Cocoate | Cognis |
| Cegesoft^{®} C24 | Ethylhexyl Palmitate | Cognis |
| Controx^{®} KS | Tocopherol, Hydrogenated Palm Glycerides Citrate | Cognis |
| Cutina^{®} CP | Cetyl Palmitate | Cognis |
| Cutina^{®} GMS-V | Glyceryl Stearate | Cognis |
| Dehyquart^{®} A CA | Aqua (Water), Cetrimonium Chloride | Cognis |
| Dehyquart^{®} F 75 | Distearoylethyl Hydroxyethylmonium Methosulfate, Cetearyl Alcohol | Cognis |
| Dow Corning^{®} 1401 Fluid | Cyclomethicone, Dimethiconol | Dow Corning |
| Dow Corning^{®} 949 | Amodimethicone, Cetrimonium Chloride, Trideceth-12 | Dow Corning |
| Dry Flo^{®} Plus | Aluminium Starch Octenylsuccinate | National Starch |
| DS-HCN^{®} | Water, Dimethylsilanol Hyaluronate | Exsymol |
| Emulmetik^{®} 100 | Lecithin | Degussa |
| Eumulgin^{®} B 2 | Ceteareth-20 | Cognis |
| Euperlan^{®} PK 3000 AM | Aqua /(Water), Glycol Distearate, Glycerin, Laureth-4, Cocamidopropyl Betaine, Formic Acid | Cognis |
| Gafquat^{®} 755N | Polyquaternium-11 | ISP |
| Gatuline^{®} R/C | Fagus Silvatica (Beech Tree Buds) Extract | Gattefosse |
| Gluadin^{®} W 20 | Aqua (Water), Hydrolyzed Wheat Protein, Sodium Benzoate, Phenoxyethanol, Methylparaben, Propylparaben | Cognis |
| Gluadin^{®} WLM | Hydrolyzed Wheat Protein | Cognis |
| Gluadin^{®} WQ | Aqua (Water), Laurdimonium Hydroxypropyl Hydrolyzed Wheat Protein, Ethylparaben, Methylparaben | Cognis |
| Luviskol^{®} K 30 | PVP | BASF |
| Montanov^{®} 68 | Cetearyl Alcohol and Cetearyl Glucoside | Seppic |
| Myritol^{®} 318 | Caprylic/Capric Triglyceride | Cognis |
| Natipide^{®} 2 PG | Aqua (and) Lecithin (and) Propylene Glycol | Nattermann |
| Natrosol^{®} 250 HR | Hydroxyethylcellulose | Hercules |
| Neo Heliopan^{®} AP | Menthyl Anthranilate | Haarmann & Reimer |
| Neutrol^{®} TE | Tetrahydroxypropyl Ethylenediamine | BASF |
| Novata^{®} AB | Cocoglycerides | Cognis |
| Parsol^{®} SLX | Polysilicone-15 | Hoffman LaRoche |
| Parsol^{®} HS | Phenylbenzimidazole Sulfonic Acid | Hoffman LaRoche |
| Parsol^{®} 340 | Octocrylene | Hoffman LaRoche |
| Parsol^{®} 1789 | Butyl Methoxy-Dibenzoyl-Methane | Hoffman LaRoche |
| Polymer JR 400^{®} | Polyquaternium-10 | Amerchol |
| Rheocare^{®} CTH (E) | Polyquaternium-37, Propylene Glycol Dicaprylate/Dicaprate, PPG-1 Trideceth-6 | CRL Cosmetic Rheologies |
| Salcare SC 96 | Polyquatemium-37, Propylene Glycol Dicaprylate/Dicaprate, PPG-1 Trideceth-6 | CIBA |
| Sepigel 305 | Polyacrylamide, C13-14 Isoparaffin, Laureth-7 | Seppic |
| Stenol^{®} 1618 | Cetearyl Alcohol | Cognis |
| Synthalen^{®} K | Carbomer | 3V Sigma |
| Tego^{®} Carbomer | Carbomer | Goldschmidt |
| Tego Care^{®} 150 | Glyceryl Stearate, Steareth-25, Ceteth-20, Stearyl Alkohol | Goldschmidt |
| Timiron^{®} Super Silver | Mica, Cl 77891 (Titanium Dioxide) | Merck |
| Trilon^{®} A | Trisodium EDTA | BASF |
| Unititamer^{®} T-40 | Isoeicosane | Induchem AG |
| Uvinul^{®} MBC 95 | Methylbenzylidene Camphor | BASF |
| Varisoft^{®} W 75 PG | Quaternium-87, Propylene Glycol | Goldschmidt |

### 2.1 UV-Schutz-Emulsionen

| | 1 | 2 | 3 | 4 | 5 | 6 |
|---|---|---|---|---|---|---|
| Montanov 68 | 5,00 | 5,00 | 5,00 | 5,00 | 5,00 | 5,00 |
| Myritol316 | 5,00 | 5,00 | 5,00 | 5,00 | 5,00 | 5,00 |
| Cetiol SB 45 | 0,50 | 0,50 | 0,50 | 0,50 | 0,50 | 0,50 |
| Novata AB | 2,00 | 2,00 | 2,00 | 2,00 | 2,00 | 2,00 |
| Stenol 1618 | 1,00 | 1,00 | 1,00 | 1,00 | 1,00 | 1,00 |
| Baysilon M 350 | 0,50 | 0,50 | 0,50 | 0,50 | 0,50 | 0,50 |
| Tocoperylacetat | 0,50 | 0,50 | 0,50 | 0,50 | 0,50 | 0,50 |
| Controx KS | 0,25 | 0,25 | 0,25 | 0,25 | 0,25 | 0,25 |
| Parsol SLX | - | - | 4,00 | 4,00 | - | - |
| Parsol HS | - | - | 2,00 | 2,00 | 2,00 | - |
| Neo Heliopan AP | - | - | - | - | 1,00 | - |
| Parsol 340 | - | - | - | - | 5,00 | - |
| Uvinul MBC 95 | - | 2,00 | - | - | - | - |
| Parsol 1789 | - | 1,00 | 1,80 | 1,80 | - | - |
| Propylparaben | 0,20 | 0,20 | 0,20 | 0,20 | 0,20 | 0,20 |
| Tego Carbomer | 0,50 | 0,50 | 0,50 | 0,50 | 0,50 | 0,50 |
| Hexandiol | 6,00 | 6,00 | 6,00 | 3,00 | 6,00 | - |
| Talkum Pharam G | 0,50 | 0,50 | 0,50 | 0,50 | 0,50 | 0,50 |
| Methylparaben | 0,20 | 0,20 | 0,20 | 0,20 | 0,20 | 0,20 |
| Glycerin | 4,50 | 4,50 | 4,50 | 3,00 | 4,50 | 3,00 |
| Dry Flo Plus | 1,00 | 1,00 | 1,00 | 1,00 | 1,00 | 1,00 |
| Natipide 2 PG | 1,00 | 1,00 | 1,00 | 1,00 | 1,00 | 1,00 |
| Gatuline R/C | 2,00 | 2,00 | 2,00 | 2,00 | 2,00 | 2,00 |
| Hydrolysiertes Protein | 5,00 | 5,00 | 5,00 | 5,00 | 5,00 | 5,00 |
| DS-HCN | 2,00 | 2,00 | 2,00 | 2,00 | 2,00 | 2,00 |
| Trilon A | 0,10 | 0,10 | 0,10 | 0,10 | 0,10 | 0,10 |
| Phenoxyethanol | 0,40 | 0,40 | 0,40 | 0,40 | 0,40 | 0,40 |
| biomimetische Melaninpigmente | 0,02 | 0,02 | 0,10 | 0,05 | 0,001 | 0,50 |
| Parfum | 0,40 | 0,40 | 0,40 | 0,40 | 0,40 | 0,40 |
| Aqua | ad. 100 | ad. 100 | ad. 100 | ad. 100 | ad. 100 | ad. 100 |

### 2.2 Make-Up

| | 1 | 2 | 3 |
|---|---|---|---|
| TegoCare 150 | 2,20 | 2,20 | 2,20 |
| Stenol 1618 | 1,75 | 1,75 | 1,75 |
| Wacker-Belsil SDM 6022 | 0,25 | 0,25 | 0,25 |
| Cetiol 868 | 3,00 | 3,00 | 3,00 |
| Cegesoft C24 | 3,80 | 3,80 | 3,80 |
| Unititamer T-40 | 5,00 | 5,00 | 5,00 |
| Myritol316 | 5,00 | 5,00 | 5,00 |
| Glycerin | 1,00 | 1,00 | 1,00 |
| Butylen Glycol | 3,00 | 3,00 | 3,00 |
| Tego Carbomer | 0,20 | 0,20 | 0,20 |
| Fe₂O₃ (Braun 70) | 0,70 | 0,70 | 0,70 |
| Fe₂O₃ (Braun 75) | 0,25 | 0,25 | 0,25 |
| Fe₂O₃ (Gelb 10) | 0,10 | 0,10 | 0,10 |
| Fe₂O₃ (Rot 30) | 0,02 | 0,02 | 0,02 |
| biomimetische Melaninpigmente | 0,02 | 0,10 | 0,50 |
| Phenoxyethanol | 0,40 | 0,40 | 0,40 |
| Methylparaben | 0,20 | 0,20 | 0,20 |
| Propylparaben | 0,20 | 0,20 | 0,20 |
| Parfum | 0,40 | 0,40 | 0,40 |
| Aqua | ad. 100 | ad. 100 | ad. 100 |

### 2.3 Shampoo

| | Gew.-% |
|---|---|
| Citronensäure, wasserfrei | 0,6 |
| Sodium Laurethsulfate | 12,75 |
| Disodium Cocoamphodiacetate | 7,8 |
| Salicylsäure | 0,2 |
| D-Panthenol 75 % | 0,2 |
| Natriumbenzoat | 0,5 |
| Euperlan PK 3000 AM | 2,6 |
| Cetiol HE | 0,5 |
| Hydrogenated Castor Oil | 0,1 |
| Polymer JR 400 | 0,2 |
| Parfum | 0,4 |
| PEG-40 Hydrogenated Castor Oil 455 | 1,0 |
| Natriumchlorid | 0,5 |
| biomimetische Melaninpigmente | 0,2 |
| Wasser | ad 100 |

### 2.4 Aufbaucreme

| | Gew.-% |
|---|---|
| Paraffinum Liquidum | 1,5 |
| Dehyquart F 75 | 1,5 |
| Isopropylmyristat | 1,0 |
| Varisoft W 575 PG | 1,5 |
| Cetearyl Alcohol | 3,5 |
| Emulmetik 100 | 0,4 |
| Propylparaben | 0,15 |
| Cutina CP | 0,7 |
| Stearamidopropyldimethylamine | 0,8 |
| Dehyquart A CA | 3,0 |
| Citronensäure, wasserfrei | 0,4 |
| Methylparaben | 0,15 |
| Phenoxyethanol, rein | 0,4 |
| D-Panthenol 75 % | 0,2 |
| Salcare SC 96 | 0,5 |
| biomimetische Melaninpigmente | 0,1 |
| Wasser | ad 100 |

### 2.4 Direct Repair für das Haar

| | Gew.-% |
|---|---|
| Synthalen K | 0,25 |
| Sepigel 305 | 2,6 |
| Dehyquart F 75 | 0,5 |
| Polymer JR 400 | 0,3 |
| Neutrol TE | 0,4 |
| Gluadin W 20 | 1,0 |
| D-Panthenol 75 % | 0,2 |
| Luviskol K 30 Pulver | 0,1 |
| Gafquat 755N | 0,5 |
| Dow Corning 1401 Fluid | 1,0 |
| Ethanol, 96 % | 15,0 |
| biomimetische Melaninpigmente | 0,1 |
| Wasser, vollentsalzt | ad 100 |

### 2.5 Haarkur

| | Gew.-% |
|---|---|
| Varisoft W 575 PG | 1,5 |
| Paraffinum Liquidum | 1,0 |
| Dehyquart F 75 | 1,5 |
| Stearamidopropyldimethylamine | 1,0 |
| Isopropylmyristat | 2,0 |
| Cetearyl Alcohol | 5,0 |
| Cutina GMS-V | 1,0 |
| Citronensäure, wasserfrei | 0,3 |
| Dehyquart A CA | 5,0 |
| Salcare SC 96 | 0,5 |
| D-Panthenol 75 % | 0,2 |
| Gluadin W 20 | 1,0 |
| biomimetische Melaninpigmente | 0,1 |
| Wasser | ad 100 |

### 2.6 Haarspitzenfluid

| | Gew.-% |
|---|---|
| Brij 30 | 1,5 |
| Natrosol 250 HR | 1,0 |
| Dow Corning 1401 Fluid | 50,0 |
| Abil Quat 3270 | 4,0 |
| Methylparaben | 0,1 |
| Gluadin W 20 | 1,0 |
| D-Panthenol 75 % | 0,2 |
| biomimetische Melaninpigmente | 0,05 |
| Wasser, vollentsalzt | ad 100 |

### 2.7 Aromatiefenpflege

| | Gew.-% |
|---|---|
| Varisoft W 575 PG | 2,5 |
| Isopropylmyristat | 1,0 |
| Cutina GMS-V | 0,8 |
| Dehyquart F 75 | 1,0 |
| Cetearyl Alcohol | 5,1 |
| Eumulgin B 2 | 0,6 |
| Timiron Super Silver | 0,2 |
| Citronensäure Monohydrat Standard | 0,55 |
| Stearamidopropyldimethylamine | 0,8 |
| Gluadin WQ | 0,25 |
| Rheocare CTH (E) . | 0,6 |
| Methylparaben | 0,2 |
| Pantolacton | 0,2 |
| biomimetische Melaninpigmente | 0,15 |
| Dow Corning 1403 Fluid | 2,0 |
| Dow Corning 949 | 0,2 |
| Gluadin WLM | 0,25 |
| Phenoxyethanol | 0,4 |
| Wasser | ad 100 |

## Patentansprüche

1. Verfahren zur Herstellung biomimetischer Melaninpigmente, in dem in einem Reaktionsmedium mindestens ein Indolinderivat, ausgewählt aus 5,6-Dihydroxyindolin bzw. deren physiologisch verträglichen Salzen, gemeinsam mit mindestens einem Phenylalaninderivat, ausgewählt aus 3,4-Dihydroxyphenylalanin bzw. dessen physiologisch verträgliches Salz, in Gegenwart mindestens eines Oxidationsmittels umgesetzt wird.

2. Verfahren nach Anspruch 1, **dadurch gekennzeichnet, daß** als Oxidationsmittel mindestens ein Vertreter aus der Gruppe, die gebildet wird aus Sauerstoff, Metallperoxiden, Persäuren sowie anorganischen Oxidationsmitteln eingesetzt wird ist.

3. Verfahren nach einem der Ansprüche 1 oder 2, **dadurch gekennzeichnet, daß** die Indolinderivate und die Phenylalaninderivate in einem Molverhältnisbereich von 2 zu 3 bis 9 zu 1, besonders bevorzugt von 4 zu 1 bis 3 zu 2, in dem erfindungsgemäßen Verfahren eingesetzt werden.

4. Verfahren nach einem der Ansprüche 1 bis 3, **dadurch gekennzeichnet, daß** der pH-Wert des Reaktionsmediums während des Verfahrens pH 7 bis pH 12, besonders bevorzugt pH 8 bis pH 10, beträgt.

5. Biomimetische Melaninpigmente, welche nach dem Verfahren gemäß einem der Ansprüche 1 bis 4 hergestellt wurden.

6. Mittel, das in einem kosmetischen Träger Melaninpigmente enthält, welche nach dem Verfahren gemäß einem der Ansprüche 1 bis 4 hergestellt wurden.

7. Mittel nach Anspruch 6, **dadurch gekennzeichnet, daß** es zusätzlich mindestens eine weitere farbverändernde Komponente enthält.

8. Mittel nach einem der Ansprüche 6 oder 7, **dadurch gekennzeichnet, daß** es zusätzlich mindestens einen weiteren UV-Lichtschutzfilter enthält.

9. Mittel nach einem der Ansprüche 6 bis 8, **dadurch gekennzeichnet, daß** es zusätzlich mindestens ein Tensid enthält.

10. Verwendung von Melaninpigmenten, welche nach dem Verfahren gemäß einem der Ansprüche 1 bis 4 hergestellt wurden, als UV-Lichtschutzfilter.

11. Verwendung von Melaninpigmenten, welche nach dem Verfahren gemäß einem der Ansprüche 1 bis 4 hergestellt wurden, als farbverändernde Komponente, insbesondere als Farbstoff zum Färben keratinhaltiger Fasern, insbesondere menschlicher Haut oder Humanhaar.

12. Verwendung von Melaninpigmenten, welche nach dem Verfahren gemäß einem der Ansprüche 1 bis 4 hergestellt wurden, als Antioxidans.

## Claims

1. A method for preparing biomimetic melanin pigments, in which, in a reaction medium, at least one indoline derivative, selected from 5,6-dihydroxy-indoline or its physiologically acceptable salts, is reacted together with at least one phenylalanine derivative, selected from 3,4-dihydroxyphenylalanine or its physiologically acceptable salt; in the presence of a least one oxidation agent.

2. The method according to claim 1, **characterized in that** at least one representative which is formed from the group of oxygen, metal peroxides, peracids as well as inorganic oxidation agents, is applied as an oxidation agent.

3. The method according to one or claims 1 and 2, **characterized in that** the indoline derivatives and the phenylalanine derivatives are applied in a molar ratio range from 2:3 to 9:1, more preferably from 4:1 to 3:2 in the method according to the invention.

4. The method according to one of claims 1 to 3, **characterized in that** the pH value of the reaction medium during the method is from pH 7 to pH 12, more preferably pH 8 to pH 10.

5. Biomimetic melanin pigments, which were prepared in accordance with the method according to one claims of 1 to 4.

6. Agent, which contains melanin pigments in a cosmetic carrier, which were prepared in accordance with the method according to one claims 1 to 4.

7. The agent according to claim 6, **characterized in that** it further contains at least one further color-modifying component.

8. The agent according to one of claims 6 or 7, **characterized in that** it further contains at least one further UV-light protection filter.

9. The agent according to one of claims 6 to 8, **characterized in that** it further contains at least one surfactant

10. The use of melanin pigments, which were prepared according to one of claims 1 to 4, as UV-light protection filters.

11. The use of melanin pigments, which were prepared according to one of claims 1 to 4, as a color-modifying component, in particular as a dye for dyeing keratinous fibers, in particular human skin or human hair.

12. The use of melanin pigments, which were prepared according to one of claims 1 to 4, as an antioxidant.

## Revendications

1. Procédé pour la préparation de pigments biomimétiques de mélanine, dans lequel on fait réagir, dans un milieu de réaction, un dérivé d'indoline choisi parmi la 5,6-dihydroxyindoline, respectivement ses sels physiologiquement acceptables, conjointement avec au moins un dérivé de phénylalanine, choisi parmi la 3,4-dihydroxyphénylalanine, respectivement son sel physiologiquement acceptable, en présence d'au moins un agent d'oxydation.

2. Procédé selon la revendication 1, **caractérisé en ce qu'**on met en oeuvre, à titre d'agent d'oxydation, au moins un représentant du groupe formé par l'oxygène, des peroxydes métalliques, des peracides ainsi que des agents d'oxydation inorganiques

3. Procédé selon l'une quelconque des revendications 1 ou 2, **caractérisé en ce qu'**on met en oeuvre les dérivés d'indoline et les dérivés de phénylalanine dans une plage de rapports molaires de 2 - 3 à 9 - 1, de manière particulièrement préférée de 4 - 1 à 3 - 2, dans le procédé selon l'invention.

4. Procédé selon l'une quelconque des revendications 1 à 3, **caractérisé en ce que** la valeur du pH du milieu de réaction, au cours du procédé s'élève de 7 à 12, de manière particulièrement préférée de 8 à 10.

5. Pigments biomimétiques de mélanine que l'on prépare conformément au procédé selon l'une quelconque des revendications 1 à 4.

6. Agent, qui contient, dans un support cosmétique, des pigments de mélanine que l'on prépare conformément au procédé selon l'une quelconque des revendications 1 à 4.

7. Agent selon la revendication 6, **caractérisé en ce qu'**il contient en outre au moins un composant supplémentaire modifiant les couleurs.

8. Agent selon la revendication 6 ou 7, **caractérisé en ce qu'**il contient en outre au moins un filtre supplémentaire de protection contre la lumière UV.

9. Agent selon l'une quelconque des revendications 6 à 8, **caractérisé en ce qu'**il contient en outre au moins un agent tensioactif.

10. Utilisation de pigments de mélanine, que l'on prépare conformément au procédé selon l'une quelconque des revendications 1 à 4, à titre de filtre de protection contre la lumière UV.

11. Utilisation de pigments de mélanine, que l'on prépare conformément au procédé selon l'une quelconque des revendications 1 à 4, à titre de composant modifiant les couleurs, en particulier à titre de colorant pour la teinture de fibres kératiniques, en particulier de la peau humaine ou des cheveux humains.

12. Utilisation de pigments de mélanine, que l'on prépare conformément au procédé selon l'une quelconque des revendications 1 à 4, à titre d'antioxydant.
